**Europäisches Patentamt**

**European Patent Office**

**Office européen des brevets**

(19)

(11) Veröffentlichungsnummer: **0 019 199**
**B1**

(12)

# EUROPÄISCHE PATENTSCHRIFT

(45) Veröffentlichungstag der Patentschrift:
23.01.85

(51) Int. Cl.⁴: **A 61 B 6/00, A 61 F 5/42**

(21) Anmeldenummer: **80102481.1**

(22) Anmeldetag: **07.05.80**

(54) **Hilfsgerät mit Kontrastmittel-Injektor für die retrograde Urethrographie.**

(30) Priorität: **09.05.79 DE 2918570**
**09.05.79 DE 7913314 U**

(43) Veröffentlichungstag der Anmeldung:
**26.11.80 Patentblatt 80/24**

(45) Bekanntmachung des Hinweises auf die Patenterteilung:
**23.01.85 Patentblatt 85/4**

(84) Benannte Vertragsstaaten:
**AT BE CH DE FR GB IT LI LU NL SE**

(56) Entgegenhaltungen:
**DE - A - 2 816 391**
**DE - C - 562 218**
**FR - A - 1 432 705**
**GB - A - 1 497 441**
**US - A - 3 631 853**
**US - A - 3 888 234**

**JOURNAL OF THE EXPERIMENTAL ANALYSIS OF BEHAVIOR, Band 8, Nr. 3, Mai 1965, Seiten 169-170 Ann Arbor, U.S.A. K. FREUND et al.: "A simple transducer for mechanical plethysmography of the male genital"**

(73) Patentinhaber: **Beiersdorf Aktiengesellschaft,**
**Unnastrasse 48, D-2000 Hamburg 20 (DE)**

(72) Erfinder: **Hübner, Egon, Hanstedter Strasse 8,**
**D-2116 Asendorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf ein Hilfsgerät mit Kontrastmittel-Injektor und Einrichtung zur Streckung und Festlegung des Penis im Strahlengang bei retrograder Urethrographie, das heißt bei der röntgenographischen Darstellung der von außen mit Röntgenkontrastmittel beschickten Harnröhre.

Nach den bisherigen Methoden war es schwierig, einmal das Röntgenkontrastmittel einwandfrei und schmerzlos in die Harnröhre des Patienten einzubringen und dort zu halten und andererseits den Penis im Strahlengang der Röntgenapparatur ohne schattengebende Hilfsinstrumente zu strecken und festzulegen.

Bei einer dieser Methoden wird zum Beispiel das Kontrastmittel über einen Katheter in die Harnröhre eingeführt, wobei eine aufblähbare Blockermanschette zur Abdichtung verwendet wird. Es besteht dabei die Gefahr der Verletzung der Harnröhre durch zu starkes Aufblähen der Manschette; bei weniger starkem Aufblähen der Manschette ist ein häufiges Herausrutschen des Katheters aus der Harnröhre beim Einbringen des Kontrastmittels nicht immer zu vermeiden.

Das manuelle Einbringen des Penis in den Strahlengang und das Festlegen des Penis im Strahlengang durch die die Untersuchung durchführende Person ist eine bekannte Methode. Bei dieser Methode ist es jedoch schwierig, die Hand oder die Finger der die Untersuchung durchführenden Person außerhalb des Strahlengangs zu halten, was eine erhebliche Gefährdung darstellt und mit den geltenden Strahlenschutzverordnungen nicht vereinbar ist. Dabei Schutzhandschuhe, wie z. B. Bleihandschuhe, zu verwenden, ist sehr nachteilhaft, da die Handschuhe schattengebend sind und das Halten mit den Schutzhandschuhen für den Patienten schmerzhaft ist.

Eine andere Methode, wie sie beispielsweise in der US-A-3 888 234 beschrieben ist, nämlich den Penis mit einer Klemme zu umfassen, die dann mit zwei oder mehr Zügeln für eine Streckung sorgt und eine Halterung darstellt, ist nicht geeignet, in allen Bereichen der Harnröhre einwandfreie Untersuchungen durchzuführen, da die Klemmen meist aus Metall und somit schattengebend sind, was Veränderungen des Lumen der Harnröhre im Klemmenbereich zur Folge hat. Auch das bei dieser Methode praktizierte Einführen des Kontrastmittels mittels einer anzusetzenden Olive ist mit Handhabungsschwierigkeiten behaftet, da die Halterung keine stabile Führung nach dem Aufsetzen der Olive gewährleistet. Außerdem wird die Streckung des Penis mittels einer Klammervorrichtung vom Patienten als sehr unangenehm empfunden.

Aus der FR-A-1 432 705 ist ein Gerät zur Kontrastmitteleingabe für eine röntgenologische Untersuchung des Uterus bekannt, welche mit Unterdruck arbeitet. Die organspezifische Formgebung der Kuppel ist jedoch ausschließlich zur Adaption an den Muttermund bestimmt und ein abdichtendes Element in Form einer Manschette ist dabei nicht erforderlich.

Gegenstand der DE-C-562 218 und der US-A-3 631 853 sind speziell ausgebildete Instrumente zur Behandlung des Penis, welche zwar ebenfalls mit einem gewissen Unterdruck arbeiten, jedoch im übrigen völlig ungeeignet sind zur Streckung und Festlegung des Penis im Strahlengang bei der Urethrographie und zur Kontrastmittelinjektion in die Harnröhre.

Es ist deshalb Aufgabe der Erfindung, die vorbeschriebenen Nachteile durch ein Hilfsgerät zu vermeiden, das eine Gefährdung der zu untersuchenden Person ausschließt, das problemlose Einbringen des Kontrastmittels sowie die Halterung und die Führung des zu untersuchenden Penis im Strahlengang gewährleistet und für den Patienten angenehmer ist sowie jegliche Verletzungsmöglichkeit entfallen läßt.

Zur Lösung dieser Aufgabe wird ein Hilfsgerät der eingangs genannten Art bereitgestellt, das gekennzeichnet ist durch einen zur Aufnahme und Halterung des Penis ausgebildeten Zylinder, in dem ein Unterdruck erzeugbar ist und der eine offene Stirnseite für die Einführung des Penis und eine geschlossene Stirnseite mit einem Durchlaß, vorzugsweise mit einer abdichtenden Führungsbuchse, für ein in das Innere des Zylinders führendes Verbindungsrohr aufweist, und ferner gekennzeichnet ist durch eine am Penis mittels des Unterdrucks festlegbare, aus elastischem Material bestehende Manschette, die im Bereich des offenen Endes des Zylinders angebracht ist und über den zu untersuchenden Penis streifbar ist, so daß die Manschette nach Erzeugung von Unterdruck im Zylinder dicht am Penis anliegt und den Innenraum des Zylinders zwischen Penis und Zylinderwandung abdichtet.

Vorteilhafte Ausgestaltungen dieses Hilfsgeräts sind in den Ansprüchen 2 bis 10 gekennzeichnet.

Der Zylinder des Gerätes besteht vorteilhafterweise aus einem eine Verschattung im Strahlengang ausschließenden transparenten Werkstoff. Das Verbindungsrohr ist am vorderen Ende vorzugsweise mit einem olivenförmigen Aufsatz zum Einbringen des Kontrastmittels in die Harnröhre versehen.

Mit dem gemäß der Erfindung ausgebildeten Hilfsgerät ist gegenüber den bisherigen Methoden jegliche Verschattung im Harnröhrenbereich ausgeschlossen, eine Veränderung des Lumen entfällt, und die Führung des Penis und des Geräts ist einwandfrei zu handhaben.

Weitere Einzelheiten des gemäß der Erfindung ausgebildeten Hilfsgeräts mit Kontrastmittel-Injektor zur retrograden Urethrographie sind den in der Zeichnung dargestellten Ausführungsbeispielen, die nachfolgend im einzelnen erläutert sind, zu entnehmen. Hierbei zeigt

Fig. 1 eine Ausführungsform des Hilfsgeräts in Seitenansicht;

Fig. 2 eine Ausführungsform des Hilfsgeräts nach Fig. 1 mit zugeordnetem Ventil-System;

Fig. 3 und 4 alternative Gestaltung des Ventilsystems.

Das Gerät besteht hierbei aus einem einseitig offenen und dem Penis in seinem Innendurchmesser angepaßten Zylinder 1, z. B. aus transparentem Werkstoff. Die offene Seite 2 des Zylinders 1 besitzt einen über dessen Umfang sich erstreckenden Wulst und/oder eine Einschnürung wellenförmigen Querschnitts zur Aufnahme einer Manschette, z. B. eines kondomartigen Überzugs 16 aus elastischem Material, wie beispielsweise Latex, für die Abdichtung des Innenraumes des Zylinders zwischen Penis und Zylinderwandung 5. Die Ränder dieser Seite des Zylinders sind entsprechend ausgebildet, um Verletzungen des Patienten auszuschließen.

Die gegenüberliegende Stirnseite 3 des Zylinders 1 weist korzentrisch eine abdichtende Führung, z. B. in Form einer Stopfbuchse 4, auf, die ein axial bewegliches Verbindungsrohr 8 aufnimmt, das außerhalb des Zylinders 1 einen Absperrhahn 9 und ein Anschlußstück 10, z. B. in Form einer Luer-Lok-Bohrung, besitzt. Innenseitig des Zylinders 1 endet das Verbindungsrohr 8 in einer aufgesetzten Gummiolive 11.

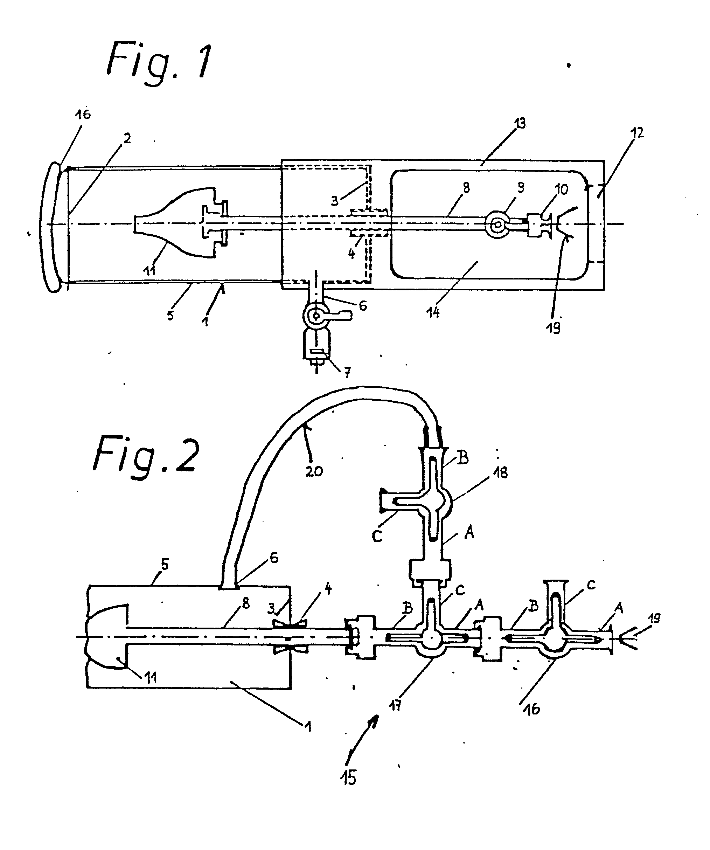

Die Außenwandung 5 des Zylinders 1 ist mit einem in den Innenraum geführten Anschluß 6 ausgestattet, der mit einem Absperrhahn mit außenliegendem Luer-Lok-Konus 7 für einen Unterdruck erzeugenden Anschluß bestückt ist oder von dem eine Leitung zu einem System von Wegeventilen (15, Fig. 2) führt, mit denen die einzelnen Phasen zur Vorbereitung der Untersuchung gesteuert werden können.

Zur Verbesserung der Handhabung des Zylinders 1 kann dieser auf eine oder in eine ebenfalls vorzugsweise zylindrische Halterung 13 geschoben und verrastet oder geschraubt werden, die in der Zylinderwand seitliche Durchbrüche 14 besitzt und an der Stirnseite 12 offen und so ausgebildet ist, daß die gegen das Anschlußstück 10 angesetzte Spritze 19 darin gehalten und geführt ist.

Es ist auch möglich, den Zylinder 1 und die Halterung 13 aus einem Stück zu fertigen. Eine weitere Variante ist möglich, indem z. B. der Zylinderraum mit einem Kolben ausgestattet wird, um dadurch einen Unterdruck in dem Teil des Zylinders 1 zu erzeugen, in den die Gummiolive 11 hineinragt. Diese und andere Varianten sind möglich, wie z. B. auch zwei übereinander greifende, einseitig mit einem Boden versehene Zylinder, mit denen ein Hub zur Evakuierung ausgeführt wird.

In Fig. 2 ist der Zylinder 1 in einer anderen ein generell mit 15 bezeichnetes System von Wegeventilen einschließenden Ausführungsform vereinfacht dargestellt. Der Anschluß 6 führt hierbei über eine Leitung 20 zu diesem vorzugsweise drei Wegeventile 16, 17, 18 einschließenden System 15.

Die Wegeventile 16, 17 und 18 sind innerhalb des Systems 15 und zu den in das Innere des Zylinders 1 führenden Anschlüssen so angeordnet, daß unter Verwendung von Dreiwegeventilen und unter Ansetzen vorzugsweise einer Spritze 19 an 16A in der ersten Phase der Evakuierung des Innenraumes des Zylinders 1 ein Durchgang vom Zylinderraum über 18B, 18A, 17C, 17A, 16B, 16A zur Spritze 19 o. ä. eingestellt ist. In der zweiten Phase der Entlüftung der Spritze 19 führt der Durchgang über 16A, 16B, 17A, 17C, 18A, 18C nach außen. Die dritte Phase des Aufziehens des an 16C anliegenden Kontrastmittels erfolgt über 16C und 16A zur Spritze 19. Schließlich erfolgt in der vierten Phase die Injektion des Kontrastmittels in die Harnröhre von der Spritze 19 über 16A, 16B, 17A, 17B und durch das Verbindungsrohr 8.

Durch Verwendung von Mehrwegeventilen läßt sich das als Ausführungsbeispiel unter Verwendung von drei Dreiwegeventilen erläuterte System 15 auch von der Anzahl der verwendeten Ventile her reduzieren, sofern eine Anordnung von einem oder ungünstigstenfalls zwei Mehrwegeventilen gegenüber der in Serie gefertigten drei Dreiwegeventile im Zusammenhang mit dem als Einwegartikel konzipierten Zylinder 1 kostengünstiger ist.

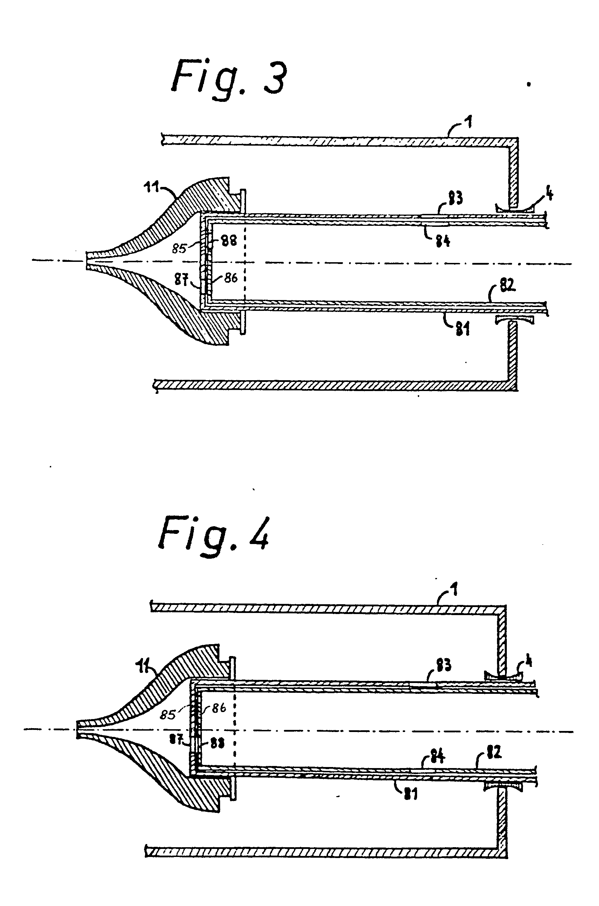

Eine weitere Möglichkeit der Evakuierung der Luft aus Zylinder 1 und der Injektion des Kontrastmittels in die Harnröhre besteht durch die in Fig. 3 und 4 dargestellte Ausführungsform des Verbindungsrohres 8.

Bei dieser Ausführungsform ist das Verbindungsrohr 8 doppelwandig ausgeführt, d. h. in einem äußeren Rohrteil 81 befindet sich ein inneres Rohrteil 82, wobei die beiden Rohrteile gegeneinander verdrehbar sind und aneinander dicht anliegen. Die beiden Rohrteile weisen je einen seitlichen Durchbruch 83 bzw. 84 auf, die durch gegenseitiges Verdrehen der beiden Rohrteile 81 und 82 zur Deckung gebracht werden können. Die relative Stellung der beiden Rohrteile zueinander, in der die beiden Durchbrüche 83 und 84 auf Deckung stehen, wird nachstehend mit Stellung A bezeichnet.

Außerdem sind die in den Zylinder 1 ragenden Enden beider Rohrteile 81 und 82 mit dicht aneinander anliegenden Stirnwänden 85 bzw. 86 verschlossen, wobei diese Stirnwände 85 und 86 je einen außerhalb der Achse der Rohrteile 81 bzw. 82 liegenden Durchbruch 87 bzw. 88 aufweisen. In Stellung A überlappen die Durchbrüche 87 bzw. 88 nicht, so daß das Innere des Rohrteils 82 mit dem Innenraum des Zylinders 1 nur über die Durchbrüche 83 und 84 verbunden ist. Die beiden Durchbrüche 87 und 88 sind so gestaltet und positioniert, daß beim Verdrehen der beiden Rohrteile 81 und 82 aus Stellung A eine Überlappung der Durchbrüche 87 und 88 erst eintritt, nachdem jede Überlappung der Durchbrüche 83 und 84 aufgehoben ist. Die relative Stellung der beiden Rohrteile zueinander, in der die beiden Durchbrüche 87 und 88 auf Deckung stehen, wird nachstehend mit Stellung B bezeichnet. Um von Stellung A in Stellung B zu gelangen, ist vorzugsweise eine Verdrehung der beiden Rohrteile 81 und 82 um 180° vorgesehen, wobei es zweckmäßig ist, durch übliche Mittel die Stellungen A und B jeweils als Endstellung auszubilden. Fig. 3 gibt Stellung A und Fig. 4 Stellung B der beiden Rohrteile wieder. In Stellung A ist es möglich, im Inneren des Zylinders 1 Unterdruck zu erzeugen. Es hat sich als ausreichend erwiesen, Unterdruck in der Größenordnung von 1 m Wassersäule anzuwenden. Nach Verdrehen der beiden Rohrteile 81 und 82 nach Stellung B kann dann Kontrastmittel durch das Innenrohr 82 und die in den Ausgang der Harnröhre geführte Olive 11 in die Harnröhre gespritzt werden.

Bei der in Fig. 3 und 4 wiedergegebenen Ausführungsform genügt ein mit dem Rohrteil 82 verbundenes Dreiwegeventil (in Fig. 3 und 4 nicht dargestellt), das das Rohrteil 82 in Stellung A mit einer Unterdruck erzeugenden Vorrichtung und in Stellung B mit einem Kontrastmittelreservoir zu verbinden erlaubt. Alternativ ist es möglich, die Funktion des Dreiwegeventils auch durch entsprechende weitere Durchbrüche der Rohrteile 81 und 82 (in Fig. 3 und 4 nicht dargestellt) zu erreichen, wobei ein Durchbruchpaar in Stellung A das Rohrteil 82 mit dem Unterdruck erzeugenden Mittel verbindet und ein zweites Durchbruchpaar in Stellung B das Rohrteil 82 mit dem Kontrastmittelreservoir, beispielsweise eine mit Kontrastmittel gefüllte Spritze, verbindet. Unter einem Unterdruck erzeugenden Mittel ist beispielsweise eine Vakuumpumpe oder eine ungefüllte Spritze zu verstehen.

## Patentansprüche

1. Hilfsgerät mit Röntgenkontrastmittelinjektor und Einrichtung zur Streckung und Festlegung des Penis im Strahlengang bei retrograder Urethrographie, gekennzeichnet durch einen zur Aufnahme und Halterung des Penis ausgebildeten Zylinder (1), in dem ein Unterdruck erzeugbar ist und der eine offene Stirnseite (2) für die Einführung des Penis und eine geschlossene Stirnseite (3) mit einem Durchlaß, vorzugsweise mit einer abdichtenden Führungsbuchse (4), für ein in das Innere des Zylinders (1) führendes Verbindungsrohr (8) aufweist, und ferner gekennzeichnet durch eine am Penis mittels des Unterdrucks festlegbare, aus elastischem Material bestehende Manschette (16), die im Bereich des offenen Endes des Zylinders (1) angebracht ist und über den zu untersuchenden Penis streifbar ist, so daß die Manschette (16) nach Erzeugung von Unterdruck im Zylinder (1) dicht am Penis anliegt und den Innenraum des Zylinders (1) zwischen Penis und Zylinderwandung (5) abdichtet.

2. Hilfsgerät nach Anspruch 1, dadurch gekennzeichnet, daß die Wandung (5) des Zylinders (1) mindestens einen aus dem Inneren des Zylinders (1) führenden Anschluß (6), z. B. zur Erzeugung von Unterdruck im Zylinderinnenraum, aufweist.

3. Hilfsgerät nach Anspruch 1, dadurch gekennzeichnet, daß das Verbindungsrohr (8) aus zwei gegeneinander verdrehbaren konzentrischen Rohrteilen (81, 82) besteht, die aneinander dichtend anliegen, und je einen seitlichen Durchbruch (83 bzw. 84) sowie je einen Durchbruch (87 bzw. 88) in den Stirnwänden (85 bzw. 86) aufweisen, wobei durch gegenseitiges Verdrehen der Rohrteile (81, 82) die beiden Durchbruchpaare (83, 84 bzw. 87, 88) nacheinander zur Deckung gebracht werden können.

4. Hilfsgerät nach einem der Ansprüche 1—3, dadurch gekennzeichnet, daß das Verbindungsrohr (8) axialbeweglich gegenüber dem Zylinder (1) angeordnet ist und daß an dem in den Zylinder (1) ragenden Ende eine Gummiolive (11) angebracht ist.

5. Hilfsgerät nach einem der Ansprüche 1—4, dadurch gekennzeichnet, daß das aus dem Zylinder (1) ragende Ende des Verbindungsrohres (8) einen Absperrhahn (9) und ein Anschlußstück (10) für das Ansetzen einer Spritze (19) aufweist.

6. Hilfsgerät nach einem der Ansprüche 2, 4 oder 5, dadurch gekennzeichnet, daß der aus dem Inneren des Zylinders (1) führende Anschluß (6) mit einem Absperrhahn mit Anschlußstück (7) für die Erzeugung von Unterdruck im Zylinderraum ausgestattet ist.

7. Hilfsgerät nach einem der Ansprüche 1—6, dadurch gekennzeichnet, daß der Zylinder (1) an der geschlossenen Stirnseite (3) zum Aufstecken und/oder Einrasten oder Aufschrauben einer Halterung (13) einen Ansatz, vorzugsweise mit reduziertem Durchmesser, eine Rastnase, ein Gewinde oder dgl. aufweist.

8. Hilfsgerät nach Anspruch 7, dadurch gekennzeichnet, daß die Halterung (13) zylinderförmig ausgebildet ist, seitliche Durchbrüche (14) aufweist und in ihrem Innendurchmesser und der offenen, der Verbindung mit dem Zylinder (1) abgekehrten Stirnseite (12) den Abmessungen der zu verwendenden Spritze (19) zu deren Führung und Halterung angepaßt ist.

9. Hilfsgerät nach einem der Ansprüche 2 oder 4—8, dadurch gekennzeichnet, daß die Verbindungen zum Inneren des Zylinders (1) aus einem der Injektion dienenden Verbindungsrohr (8) und einer dem Anschluß (6) zugeordneten Leitung (20) bestehen, die den Arbeitsphasen entsprechend mittels eines Ventilsystems (15) zu- und abgeschaltet werden.

10. Hilfsgerät nach Anspruch 9, dadurch gekennzeichnet, daß das Ventilsystem (15) aus Wegeventilen (16, 17, 18) gebildet ist, die in ihrer Zuordnung zueinander und zu den Anschlüssen (6, 8) und bei der Verwendung einer Spritze (19) derart angeordnet sind, daß das Evakuieren des Zylinders (1) über einen Ventileingang (18B), das Entlüften der Spritze (19) über einen Ventilausgang (18C), das Aufziehen des Kontrastmittels über einen weiteren Ventileingang (16C) und die Injektion in die Harnröhre über einen weiteren Ventilausgang (17B) erfolgen.

## Claims

1. Auxiliary apparatus with injector of radio-opaque fluid and device for extending and immobilising the penis in the ray path for retrograde urethrography, characterised by a cylinder (1) which is designed to receive and hold the penis and in which a subatmospheric pressure can be produced an which has an open end (2) for the introduction of the penis and a closed end (3) having a passage, preferably having a sealing and guiding socket (4), for a connecting tube (8) leading into the interior of the cylinder (1), and further characterised by a cuff (16) which can be immobilised on the penis by means of the subatmospheric pressure, is made of elastic material and is attached in the region of the open end of the cylinder (1), and which can be slipped over the penis which is to be examined so that, after subatmospheric pressure has been produced in the cylinder (1), the cuff (16) sits close to the penis and seals the interior of the cylinder (1) between the penis and the cylinder wall (5).

2. Auxiliary apparatus according to claim 1, characterised in that the wall (5) of the cylinder (1) has at least one connector (6) leading out of the interior of the cylinder (1) for the production of, for example, subatmospheric pressure in the interior of the cylinder.

3. Auxiliary apparatus according to claim 1, characterised in that the connecting tube (8) comprises two concentric tubular parts (81, 82) which can be rotated with respect to one another, sit close to one another and each have a lateral perforation (83 and 84 respectively), and each have a perforation (87 and 88 respectively) in the end walls (85 and 86 respectively), it being possible, by rotating the tubular parts (81, 82) with respect to one another, to bring the two pairs of perforations (83, 84 and 87, 88) consecutively to coincidence.

4. Auxiliary apparatus according to one of claims 1—3, characterised in that the connecting tube (8) is arranged so as to be axially movable with respect to the cylinder (1), and in that a rubber olive (11) is attached to the end projecting into the cylinder (1).

5. Auxiliary apparatus according to one of claims 1—4, characterised in that the end of the connecting tube (8) projecting out of the cylinder (1) has an isolating valve (9) and a connector (10) for attaching a syringe (19).

6. Auxiliary apparatus according to one of claims 2, 4 or 5, characterised in that the connector (6) leading out of the interior of the cylinder (1) is equipped with an isolating valve having a connector (7) for the production of subatmospheric pressure inside the cylinder.

7. Auxiliary apparatus according to one of claims 1—6, characterised in that the cylinder (1) has, at hte closed end (3), an attachment, preferably with a reduced diameter, a projecting catch, a screw therad or the like, for slipping on and/or snapping on or screwing on a holding device (13).

8. Auxiliary apparatus according to claim 7, characterised in that the holding device (13) is designed in the form of a cylinder, has lateral perforations (14) and its internal diameter and open end (12), which is opposite to the connection with the cylinder (1), have dimensions appropriate for guiding and holding the syringe (19) used.

9. Auxiliary apparatus according to one of claims 2 or 4—8, characterised in that the connections to the interior of the cylinder (1) comprise a connecting tube (8), which serves for injection, and a line (20), which is assigned to connector (6), which are switched on and off according to the operating phases by means of a valve system (15).

10. Auxiliary apparatus according to claim 9, characterised in that the valve system (15) is formed of directional control valves (16, 17, 18) which are arranged with respect to one another and to the connectors (6, 8), and when a syringe (19) is used, in such a manner that the evacuation of the cylinder (1) takes place via a valve inlet (18B), the venting of the syringe (19) takes place via a valve outlet (18C), the aspiration of the radio-opaque fluid takes place via a further valve inlet (16C), and the injection into the urethra takes place via a further valve outlet (17B).

**Revendications**

1. Appareil auxiliaire à injecteur de substance de contraste et dispositif pour étirer et immobiliser le pénis dans le trajet des rayons lors d'une urétrographie rétrograde, caractérisé par un cylindre (1) d'une configuration lui permettant de recevoir et de maintenir le pénis, dans lequel une dépression peut être produite et qui présente un côté d'about (2) ouvert pour l'introduction du pénis et un côté d'about (3) fermé comportant un passage, de préférence muni d'une douille de guidage d'étanchéité (4), destiné à un tuyau de raccordement (8) s'ouvrant à l'intérieur du cylindre (1), et caractérisé, en outre, par une manchette (16) en matière élastique pouvant être fixée sur le pénis au moyen de la dépression qui est montée dans la zone de l'extrémité ouverte du cylindre (1) et qui peut être glissée sur le pénis à examiner de sorte que la manchette (16), après production de la dépression dans le cylindre (1), s'applique étroitement contre le pénis et assure l'étanchéité de l'espace intérieur du cylindre (1), entre le pénis et la paroi (5) du cylindre.

2. Appareil auxiliaire suivant la revendication 1, caractérisé en ce que la paroi (5) du cylindre (1) présente au moins un raccord (6) provenant de l'intérieur du cylindre (1), par exemple pour produire de la dépression dans l'espace intérieur du cylindre.

3. Appareil auxiliaire suivant la revendication 1, caractérisé en ce que le tuyau de raccordement (8) est formé de deux tubes concentriques (81, 82) qui peuvent tourner l'un par rapport à l'autre, qui sont appliqués de manière étanche l'un contre l'autre et que présentent chacun une lumière latérale (83, 84) ainsi qu'une lumière (87, 88) dans leurs parois d'about (85, 86), de sorte qu'une rotation réciproque des tubes (81, 82) permet d'amener les deux paires de lumières (83, 84 et 87, 88) consécutivement en coïncidence.

4. Appareil auxiliaire suivant l'une quelconque des revendications 1 à 3, caractérisé en ce que le tuyau de raccordement (8) est monté de manière à pouvoir se déplacer axialement par rapport au cylindre (1) et son extrémité, qui s'étend dans le cylindre (1), porte une olive en caoutchouc (11).

5. Appareil auxiliaire suivant l'une quelconque des revendications 1 à 4, caractérisé en ce que l'extrémité du tuyau de raccordement (8), qui sort du cylindre (1), présente un robinet d'arrêt (9) et un raccord (10) pour y appliquer une seringue (19).

6. Appareil auxiliaire suivant l'une quelconque des revendications 2, 4 ou 5, caractérisé en ce que le raccord (6) partant de l'intérieur du cylindre (1) est équipé d'un robinet d'arrêt avec un raccord (7) pour la production de la dépression dans l'espace du cylindre.

7. Appareil auxiliaire suivant l'une quelconque des revendications 1 à 6, caractérisé en ce que le cylindre (1) présente, du côté d'about fermé (3), une saillie, de préférence de plus petit diamètre, un bec d'arrêt, un pas de vis ou l'équivalent, pour glisser et/ou emboîter ou visser un support (13).

8. Appareil suivant la revendication 7, caractérisé en ce que le support (13) est cylindrique, présente des ouvertures latérales (14) et est adapté quant à son diamètre intérieur et à son côté d'about (12) ouvert opposé à la jonction au cylindre (1) , aux dimensions de la seringue (19) à utiliser, en vue de la guider et de la maintenir.

9. Appareil auxiliaire suivant l'une quelconque des revendications 2 ou 4 à 8, caractérisé en ce que les raccordements à l'intérieur du cylindre (1) sont faits d'un tuyau de raccordement (8) destiné à l'injection et d'une conduite (20) associée au raccord (6) qui sont ouvertes et fermées au moyen d'un système de valves (15) d'une manière correspondant aux phases de travail.

10. Appareil auxiliaire suivant la revendication 9, caractérisé en ce que le système de valves (15) est formé de valves (16, 17, 18) qui sont disposées les unes par rapport aux autres, par rapport aux raccords (6, 8) ainsi que lors de l'utilisation d'une seringue (19) d'une manière telle que l'évacuation de l'air du cylindre (1) s'effectue par l'intermédiaire d'une entrée de valve (18B), l'évacuation de l'air de la seringue (19) s'effectue par l'intermédiaire d'une sortie de valve (18C), l'aspiration de la substance de contraste s'effectue par l'intermédiaire d'une autre entrée de valve (16C) et l'injection dans l'urètre s'effectue par l'intermédiaire d'une autre sortie de valve (17B).

Fig. 1

Fig. 2

# Fig. 3

# Fig. 4